# EUROPEAN PATENT APPLICATION

(11) **EP 4 245 153 A1**
(43) Date of publication of application: **20.09.2023**
(21) Application number: 22460015.5
(22) Date of filing: 18.03.2022
(51) Int. Cl.: A23L 33/135, A23L 33/16, A61K 9/14

(54) **DIETARY SUPPLEMENT WITH SAPONITE CLAY**

(71) Applicant: JACH INVEST Janusz Chelchowski, 10-699 Olsztyn (PL)
(72) Inventor: Chelchowski, Janusz, 10-699 Olsztyn (PL)
(74) Representative: Matyka, Malgorzata

(57) **Abstract**

A dietary supplement with saponite clay being a synergistic blend of composition in aqueous environment it is a mixture with probiotic properties consisting of crushed saponite clay powder in the range of 1,0-2.5 g, per 100 g of composition, probiotic bacteria concentrate in the range of 0,1- 2,0 g, with a concentration of bacteria in 100.0 g of finished concentrate 1×10⁹ - 1×10¹² cfu, corn starch in the range of 0,5-2,5 g.

## Description

The invention concerns a probiotic synergistic composition for dietary supplementation containing strains of bacteria of the genus *Lactobacillus.*

Saponite clay is an aluminosilicate of natural origin.

Table 1 shows a preliminary analysis of the physical and chemical characteristics of the saponite clay tested.

**Table 1. Physicochemical properties of the tested saponite clay**

| **No.** | **Name of indicators** | **Unit measures** | **Indicators** |
|---|---|---|---|
| I | Average density | kgf/m³ | 1876-2160 |
| 2 | Porosity | % | 23,13-36,82 |
| 3 | Water absorption | % | 11,79- 25,01 |
| 4 | Moisture | % | 23,33 |
| 5 | Plasticity | - | 28,4 |
| 6 | Swelling | % | 5,6 |
| 7 | Total moisture content of the air-dry sample | % | 10-12 |
| 8 | Fire resistance | °C | 1280-1300 |

The above results, indicate that saponite clay can have applications in various forms. Due to its plasticity and water absorption, it is suitable, after appropriate preparation, for forming masks. Also, the density and moisture structure indicates that saponite clay can-be used as a natural concentrate of macro- and microelements in powder form.

The composition of the tested saponite clay was also analyzed and is shown in Table 2.

**Table 2. Chemical composition of the analyzed aluminosilicate (own research).**

| **Chemical substance** | **Unit** | **Value** |
|---|---|---|
| SiO₂ | % | 41,92-54,56 |
| Ti0₂ | | 0.98-2,42 |
| Al₂O₃ | | 11,93-15,45 |
| Fe₂0₃ | | 9,36-16,22 |
| MnO | | 0,10-0,37 |
| MgO | | 7,02-13,43 |
| Cao | | 0,78-10,62 |
| Na₂O | | 0,03-4,89 |
| K₂0 | | 0,19-3,50 |
| P₂O₅ | | 0,083-0,26 |
| H₂O⁺ | | 10,4-10,95 |
| ₋H₂O⁻ | | 4,8-5,66 |

Macro- and microelements contained in saponite clay belong to biologically active substances. They are components of natural origin and serve as vital substances that regulate the physiological functions of the body. They can be divided into the following groups:
- included in the composition of bones, teeth, skin and hair - Ca, P, Mg, S, F;
- included in compounds of fundamental importance for physiological processes-Fe, Zn, Cu; Co, J;
- playing an essential role in water-electrolyte metabolism and acid-base balance - Na, K, Cl;
- that perform a variety of regulatory functions in the body - Se, Mn, Mo, Cr, Ni, V, B, Si.

The above results, indicate that saponite clay can have applications in various forms. Due to its plasticity and water absorption, it is suitable, after appropriate preparation, for forming masks. Also, the density and moisture structure indicates that saponite clay can-be used as a natural concentrate of macro- and microelements in powder form.

**Table 3, Effect of saponite clay on the growth of potentially pathogenic strains (own research).**

| Control cultures^{∗}/cultures with the addition of 1% saponite clay | Time | incubation |
|---|---|---|
| | 0h (inoculum) | 24h |
| | Jtk/mL | Jtk/mL |
| *Candida albicans* 676 | 1,4 x 10⁵ | 3,6 x 10⁷ |
| *Candida albicans* 676 + saponite clay (1%) | 1,4 x 10⁵ | 3,4 x 10⁷ |
| *Escherichia coli* 94 | 2,8 x 10⁶ | 1,1 x 10⁹ |
| *Escherichia coli* 94 + saponite clay (1%) | 2,8 x 10⁶ | 1,2 x 10⁹ |
| *Enterococcus faecalis* L | 2,6 x 10⁵ | 6,3 x 10⁸ |
| *Enterococcus faecalis* L + saponite clay (1%) | 2,6 x 10⁵ | 9,7 x 10⁸ |
| *Enterococcus faecium* T-18 | 1,2 x 10⁶ | 6,6 x 10⁸ |
| *Enterococcus faecium* T-18 + saponite clay (1%) | 1,2 x 10⁶ | 7,3 x 10⁸ |
| *Propionibacterium acnes* ATCC 6919 | 1,4 x 10⁶ | 6,6 x 10⁸ |
| *Propionibacterium acnes* ATCC 6919 + saponite clay (1%) | 1,4 x 10⁶ | 7,1 x 10⁸ |
| *Salmonella Enteritidis* KOS-64 | 1,5 x 10⁶ | 1,1 x 10⁹ |
| *Salmonella Enteritidis* KOS-64 + saponite clay (1%) | 1,5 x 10⁶ | 1,1 x 10⁹ |
| *Staphylococcus aureus koag* (+) | 1,7 x 10⁶ | 1,6 x 10⁹ |
| *Staphylococcus aureus koag* (+) + saponite clay (1%) | 1,7 x 10⁶ | 1,1 x 10⁹ |
| *Staphylococcus epidermidis* ATCC 49134 | 9,5 x 10⁵ | 1,7 x 10⁹ |
| *Staphylococcus epidermidis* ATCC 49134 + saponite clay (1%) | 9,5 x 10⁵ | 1,9 x 10⁹ |

| | | |
|---|---|---|
| *Cultures in liquid media: *Candida albicans* - Nickerson's medium; *Escherichia coli, Enterococcus faecium, Enterococcus faecalis, Salmonella Enteritidis, Staphylococcus aureus, Staphylococcus epidermidis* - Hydrolyzed milk; *Propionibacterium acnes* - PY substrate. | | |

Test saponite clay does not show direct biological activity against selected pathogens. The numbers of strains tested in control cultures as well as in cultures with saponite clay were very similar (Table 3).

Thus, the potential medicinal qualities of saponite clay are based on its composition and the composition of micro- and macroelements. As a result, saponite clay can be used as a supplement in the treatment of musculoskeletal, respiratory, cardiovascular disorders, allergies, autoimmune diseases of the skin and subcutaneous tissue, neurology or diseases of internal organs.

Saponite clay, as a source of natural micro and macroelements, can be used as a mineral feed additive for animals, a natural feed preservative, in medicine, pharmacology and cosmetology.

Due to the lack of natural biological anti-pathogen properties, the selected strains with probiotic properties, *Lactobacillus plantarum* AMT4, *Lactobacillus plantarum* AMT7 and *Lactobacillus plantarum* AMT14, were proposed. The addition of strains with probiotic properties is intended to add the physical and mechanical activities of the saponite to the biological activity derived from the bacteria.

The antibacterial efficacy of the three freeze-dried strains tested (*Lactobacillus plantarum* AMT4, *Lactobacillus plantarum* AMT7 and *Lactobacillus plantarum* AMT 14) was determined by inhibiting the growth of *Candida albicans* 676, *Escherichia coli* 94, *Enterococcus faecalis* L. strains.

Thus, the potential medicinal qualities of saponite clay are based on its composition and the composition of micro- and macroelements. Thus, saponite clay can be used as a supplement in the treatment of musculoskeletal, respiratory, cardiovascular diseases, allergies, autoimmune diseases of the skin and subcutaneous tissue, neurology or diseases of internal organs.

Saponite clay, as a source of natural micro and macroelements, can be used as a mineral feed additive for animals, a natural feed preservative, in medicine, pharmacology and cosmetology.

Due to the lack of natural biological anti-pathogen properties, the selected strains with probiotic properties, *Lactobacillus plantarum* AMT4, *Lactobacillus plantarum* AMT7 and *Lactobacillus plantarum* AMT14, were proposed. The addition of strains with probiotic properties is intended to add physical and mechanical activities of the activity saponite - PL180610.

### Example 1

Cultures were conducted in liquid media suitable for the strain. Control samples were cultures without the addition of saponite clay.

Cultures were incubated at 37°C/24h after which microbial abundance was determined on multiplication or selective media suitable for the species.

### Example 2

The antibacterial efficacy of the three freeze-dried strains tested (*Lactobacillus plantarum* AMT4, *Lactobacillus plantarum* AMT7 and *Lactobacillus plantarum* AMT14) was determined by inhibiting the growth of *Candida albicans* 676, *Escherichia coli* 94, *Enterococcus faecalis* L, *Enterococcus faecium* T-18, *Propionibacterium acnes* ATCC 6919, *Salmonella enteritidis* KOS-64, *Staphylococcus aureus coag* (+) and *Staphylococcus epidermidis* ATCC 49134 in liquid co-cultures. The cultures were conducted in liquid medium consisting of 50% MRS liquid medium and 50% liquid medium suitable for the respective potentially pathogenic strain.

Liquid media were inoculated with freeze-dried strain in such an amount that the initial cell count was 10⁹ cfu/mL (colony-forming units/mL) and active culture of pathogenic strain at 10⁵ cfu/mL (active culture of pathogenic strains was prepared by passage of strains three times in appropriate medium, incubation at 37°C/24h).

The control samples were parallel cultures of probiotic strains and pathogenic strains in monocultures, which were inoculated in the same way as in the joint cultures.

Common and monoculture cultures were incubated at 37°C/24h, 48, 72h.

After 24,48, 72 hours of incubation, a sample was taken and the abundance of potentially pathogenic microorganisms was determined using the plate method by seeding onto solid multiplication medium or selective medium suitable for the type of microorganisms.

### Example 3

In the process of making a probiotic composition of dietary suplement, the following mixture was used: saponite clay - 0.9 g; bacteria with probiotic properties - 0.1 g; concentration of bacteria with probiotic properties in 1 g of finished product - a minimum of 1 × 10⁹ cfu, filling cosmetic products.

### Microbiological media used

Liquid medium for the culture of Lactobacillus plantarum strains:
- MRS bouillon (BTL, cat. no. P - 0132)

Liquid media for culture of potentially pathogenic strains:
- *Candida albicans* - Nickerson's medium (Merck cat. no. 1.1046)
- *Escherichia coli, Enterococcus faecium, Enterococcus faecalis, Salmonella enteritidis, Staphylococcus aureus. Staphylococcus epidermidis* - Hydrolyzed milk ( 10% milk regenerated from skimmed milk powder, hydrolyzed pancreatin).
- *Propionibacterium acnes* - PYG substrate by: https://www.dsmz.de/microorganisms/medium/pdf/DSMZ_Mediurn104.pdf

Agar media for determining the number of colony-forming units of the tested strains:
- *Lactobacillus* - MRS (*Lactobacillus* agar acc.to de Man, Rogosa and Sharpe - Merck, cat. no. 1.10660.0500) -.
- *Candida albicans* - Sabouraud Dextrose Agar (Difco, cat. no. 210950)
- *Escherichia coli* - ChromogenicMedium (Biocorp cat. no. PS 121)
- *Enterococcus faecium, Enterococcus faecalis* - KAA Confirmatory LAB medium - AGAR (Biocorp cat. no. PS 123
- *Propionibacterium acnes* - PYG agar medium by: https://www.dsmz.de/microorganisms/medium/pdf/DSMZ_Mediurn104.pdf

### Microbiological media used

Liquid medium for the culture of *Lactobacillus plantarum* strains.
- MRS Bouillon (BTL, cat. no. P=0132)

Liquid media for culture of potentially pathogenic strains
- *Candida albicans* - Nickerson's medium (Merck cat. no. 1.1046)
- *Escherichia coli, Enterococcus faecium, Enterococcus faecalis, Salmonella enteritidis, Staphylococcus aureus. Staphylococcus epidermidis* - Hydrolyzed milk ( 10% milk regenerated from skimmed milk powder, hydrolyzed pancreatin).
- *Propionibacterium acnes* - PYG substrate by: https://www.dsmz.de/microorganisms/medium/pdf/DSMZ_Mediurn104.pdf

Agar media for determining the number of colony-forming units of the tested strains:
- *Lactobacillus* - MRS (Lactobacillus agar acc. to de Man, Rogosa and Sharpe - Merck, cat. no. 1.10660.0500) -.
- *Candida albicans* - Sabouraud Dextrose Agar (Difco, cat. no. 210950)
- *Escherichia coli* - ChromogenicMedium (Biocorp cat. no. PS 121)
- *Enterococcus faecium, Enterococcus faecalis* - KAA Confirmatory LAB medium - AGAR (Biocorp cat. no. PS 123
- Propionibacterium acnes - PYG agar medium by: https://www.dsmz.de/microorganisms/medium/pdf/DSMZ_Mediurn104.pdf

### Study material and methods

### 1. Study material

Products and intermediates goods:
- Saponite clay
- Potassium Sorbate, Tetrasodium Glutamate Diacetate, Lactic Acid, Phenoxyethanol, Benzoic Acid
- pharmaceutical corn starch
- pharmaceutical lactose

Lyophilized strains of potentially probiotic strains:
- *Lactobacillus plantarum* AMT4,
- *Lactobacillus plantarum* AMT7
- *Lactobacillus plantarum* AMT14

Pathogenic strains used in antibacterial activity studies
- *Candida albicans* 676,
- *Escherichia coli* 94,
- *Enterococcus faecalis* L,
- *Enterococcus faecium* T-18,
- *Propionibacterium acnes* ATCC 6919,
- *Salmonella Enteritidis* KOS-64,
- *Staphylococcus aureus coag* (+),
- *Staphylococcus epidermidis* ATCC 12229
- *Staphylococcus epidermidis* ATCC 49134
- MRS Bouillon (BTL, cat. no. P=0132)
- Liquid media for culture of potentially pathogenic strains
- *Candida albicans* - Nickerson's medium (Merck cat. no. 1.1046)
- *Escherichia coli, Enterococcus faecium, Enterococcus faecalis, Salmonella enteritidis, Staphylococcus aureus. Staphylococcus epidermidis* - Hydrolyzed milk ( 10% milk regenerated from skimmed milk powder, hydrolyzed pancreatin).
- *Propionibacterium acnes* - PYG substrate by: https://www.dsmz.de/microorganisms/medium/pdf/DSMZ_Mediurn104.pdf

Agar media for determining the number of colony-forming units of the tested strains:
- *Lactobacillus* - MRS (*Lactobacillus* agar acc. to de Man, Rogosa and Sharpe - Merck, cat. no. 1.10660.0500) -.
- *Candida albicans* - Sabouraud Dextrose Agar (Difco, cat. no. 210950)
- *Escherichia coli* - ChromogenicMedium (Biocorp cat. no. PS 121)
- *Enterococcus faecium, Enterococcus faecalis* - KAA Confirmatory LAB medium - AGAR (Biocorp cat. no. PS 123
- Propionibacterium acnes - PYG agar medium by: https://www.dsmz.de/microorganisms/medium/pdf/DSMZ_Mediurn104.pdf

### Study material and methods

### Study material

Products and intermediates goods:
- Saponite clay
- pharmaceutical corn starch
- pharmaceutical grade lactose

Lyophilized strains of potentially probiotic strains:
- *Lactobacillus plantarum* AMT4,
- *Lactobacillus plantarum* AMT7
- *Lactobacillus plantarum* AMT14

Pathogenic strains used in antibacterial activity studies
- *Candida albicans* 676,
- *Escherichia coli* 94,
- *Enterococcus faecalis* L,
- *Enterococcus faecium* T-18,
- *Propionibacterium acnes* ATCC 6919,
- *Salmonella Enteritidis* KOS-64,
- *Staphylococcus aureus coag* (+),
- *Staphylococcus epidermidis* ATCC 12229
- *Staphylococcus epidermidis* ATCC 49134

### Microbiological media used

Liquid media for the culture of *Lactobacillus plantarum* strains.

MRS bouillon (BTL, cat. no. P - 0132)

Liquid media for culture of potentially pathogenic strains
- *Candida albicans* - Nickerson's medium (Merck cat. no. 1.1046)
- *Escherichia coli, Enterococcus faecium, Enterococcus faecalis, Salmonella enteritidis, Staphylococcus aureus. Staphylococcus epidermidis* - Hydrolyzed milk ( 10% milk regenerated from skimmed milk powder, hydrolyzed pancreatin).
- *Propionibacterium acnes* - PYG substrate by: https://www.dsmz.de/microorganisms/medium/pdf/DSMZ_Mediurn104.pdf

Agar media for determining the number of colony-forming units of the tested strains:
- *Lactobacillus* - MRS (Lactobacillus agar acc. to de Man, Rogosa and Sharpe - Merck, cat. no. 1.10660.0500) -.
- *Candida albicans* - Sabouraud Dextrose Agar (Difco, cat. no. 210950)
- *Escherichia coli* - ChromogenicMedium (Biocorp cat. no. PS 121)
- *Enterococcus faecium, Enterococcus faecalis* - KAA Confirmatory LAB medium - AGAR (Biocorp cat. no. PS 123
- *Propionibacterium acnes* - PYG agar medium by: https://www.dsmz.de/microorganisms/medium/pdf/DSMZ_Mediurn104.pdf

## Claims

1. A dietary supplement with saponite clay being a synergistic blend of composition, **wherein** it is a blend with probiotic properties consisting of crushed saponite clay powder in the range of 0.2-1.0 g, per 100 g of composition, corn starch in the range of 0.5-1.0 g, probiotic bacteria concentrate in the range of 0.1- 0.2 g, with a concentration of bacteria in 100.0 g of finished concentrate 1×10⁹ - 1×10¹² cfu.

2. The composition according to claim. 1, **wherein** contains the bacteria *Lactobacillus plantarum* AMT4, *Lactobacillus plantarum* AMT7, *Lactobacillus plantarum* AMT14.

3. The composition according to claim. 1, **wherein** contains at least one type of bacteria of the genus *Lactobacillus* in an acceptable environment.

4. The composition according to claim. 1, **wherein** an aqueous mixture with a pH in the range of 6-8.
